# EUROPÄISCHE PATENTANMELDUNG

(11) **EP 0 790 313 A2**
(43) Veröffentlichungstag der Anmeldung: **20.08.1997**
(21) Anmeldenummer: 97101507.8
(22) Anmeldetag: 31.01.1997
(51) Int. Cl.: C12N 15/85, C12N 5/10, A61K 31/70, A61K 48/00

(54) **Nukleinsäurekonstrukte für die zellzyklusregulierte Expression von Genen, derartige Konstrukte enthaltende Zellen sowie deren Verwendung zur Herstellung von Heilmitteln**

(30) Priorität: 13.02.1996 DE 19605274
(71) Anmelder: HOECHST AKTIENGESELLSCHAFT, 65929 Frankfurt am Main (DE)
(72) Erfinder: Müller, Rolf, Prof.Dr., 35037 Marburg (DE); Zwicker, Jörk, Dr. University of CA at Berkeley, Berkeley, CA 94720 (US); Sedlacek, Hans-Harald, Prof.Dr., 35041 Marburg (DE)

(57) **Zusammenfassung**

Offenbart werden Nukleinsäurekonstrukte für die sowohl zellspezifische oder virusspezifische wie auch zellzyklusregulierte Expression von wenigstens einem Gen umfassend folgende Komponenten:
a) eine Aktivatorsequenz, deren Funktion die zellspezifische oder virusspezifische Aktivierung der basalen Transkription ist.
b) ein Promotormodul, das eine Nukleotidsequenz aufweist, die wenigstens ein Protein der E2F-Familie bindet und eine weitere Nukleotidsequenz aufweist, die wenigstens ein Protein der CHF-Familie bindet und die desweiteren eine die Transkription initiierende Sequenz enthalten kann.
c) ein Strukturgen, das für einen Wirkstoff kodiert, der sowohl zellspezifisch oder virusspezifisch wie auch zellzyklusabhängig exprimiert wird.

Derartige Nukleinsäurekonstrukte sind zur Verwendung in der Gentherapie geeignet.

## Beschreibung

Es werden Nukleinsäurekonstrukte beschrieben, die in der Gentechnologie und insbesondere in der Gentherapie von Erkrankungen verwendet werden können.

Bei der Gentherapie werden Gene in den Organismus eingeführt, die im Organismus exprimiert werden sollen. Die Regulation der Expression dieser Gene ist bedeutsam für den therapeutischen Effekt der Gentherapie.

Gegenstand der vorliegenden Erfindung sind daher Nukleinsäurekonstrukte, die bei der Gentherapie verwendet werden können. Hierbei handelt es sich um Nukleinsäurekonstrukte für die sowohl zellspezifische oder virusspezifische wie auch zellzyklusregulierte Expression von wenigstens einem Gen umfassend folgende Komponenten:
a) eine Aktivatorsequenz, deren Funktion die zellspezifische oder virusspezifische Aktivierung der basalen Transkription ist.
b) ein Promotormodul, das eine Nukleotidsequenz aufweist, die wenigstens ein Protein der E2F-Familie bindet und eine weitere Nukleotidsequenz aufweist, die wenigstens ein Protein der CHF-Familie bindet und die desweiteren eine die Transkription initiierende Sequenz enthalten kann.
c) ein Strukturgen, das für einen Wirkstoff kodiert, der sowohl zellspezifisch oder virusspezifisch wie auch zellzyklusabhängig exprimiert wird.

Bei den erfindungsgemäßen Nukleinsäurekonstrukten weist das Promotormodul (b) eine Nukleotidsequenz auf, die einerseits wenigstens ein Protein der E2F-Familie bindet. Diese Sequenz wird bevorzugt ausgewählt aus Nukleotidsequenzen, die die Nukleotidsequenz CTTGGCGGG oder eine funktionell ähnliche (analoge) Sequenz, wie beispielsweise TTTCGCGCC (E2F-Bindestelle des DHFR-Genes) oder TTTCGCGGC (E2F-1 Bindestelle) enthalten.

Andererseits weist das Promotormodul (b) eine Nukleotidsequenz auf, die wenigstens ein Protein der CHF-Familie bindet. Diese Sequenz enthält bevorzugterweise die Nukleotidsequenz TAGGAAA oder eine funktionell ähnliche (analoge) Sequenz.

Bei den erfindungsgemäßen Nukleinsäurekonstrukten kann das Promotormodul (b) die Wirkung der stromaufwärts gelegenen Aktivatorsequenz in der G0- und G1-Phase des Zellzyklus hemmen und dadurch die Expression des stromabwärts gelegenen Gens (c) inhibieren.

In bevorzugter Ausführungsform ist bei den erfindungsgemäßen Nukleinsäurekonstrukten die Aktivatorsequenz (a) zellspezifisch oder virusspezifisch.

Die Nukleinsäurekonstrukte bestehen bevorzugterweise aus DNS. Unter dem Begriff "Nukleinsäurekonstrukte" werden künstliche Gebilde aus Nukleinsäure verstanden, die sich in den Zielzellen transkribieren können. Sie sind bevorzugt in einen Vektor eingefügt, wobei Plasmidvektoren oder virale Vektoren besonders bevorzugt sind.

Durch die erfindungsgemäßen Nukleinsäurekonstrukte kann ein Gen (c) sowohl zellspezifisch oder virusspezifisch als auch zellzyklusspezifisch exprimiert werden, wobei es sich bei dem Gen (c) bevorzugt um ein Gen handelt, das für einen pharmakologisch aktiven Wirkstoff oder aber für ein Enzym kodiert, welches eine inaktive Vorstufe eines Pharmakons spaltet in ein aktives Pharmakon.

Die erfindungsgemäßen Nukleinsäurekonstrukte weisen eine Aktivierungssequenz (a) auf, die bevorzugt ausgewählt ist aus der Gruppe von Promotoren oder Enhancern, die die Transkription in Endothelzellen, glatte Muskelzellen, blutbildende Zellen, Lymphozyten, Makrophagen, Tumorzellen oder Gliazellen aktivieren oder aus Promotorsequenzen bzw. Enhancersequenzen der Viren HBV, HCV, HSV, HPV, EBV, HTLV oder HIV.

Gegenstand der vorliegenden Erfindung sind auch isolierte Zellen, die ein erfindungsgemäßes Nukleinsäurekonstrukt enthalten.

Bevorzugt verwendet werden die erfindungsgemäßen Nukleinsäurekonstrukte oder diese enthaltende Zellen zur Herstellung eines Hei mittels zur Behandlung einer Erkrankung, die mit übermäßiger Zellvermehrung einhergeht, wobei die Herstellung des Heilmittels die Einführung eines Nukleinsäurekonstrukts in eine Zielzelle und seine Expression in dem Stadium der Zellproliferation umfaßt.
Die erfindungsgemäßen Nukleinsäurekonstrukte kommen in dieser Form nicht in der Natur vor, d.h. das Gen (c) des Nukleinsäurekonstrukts ist nicht natürlicherweise kombiniert mit der Aktivatorsequenz (a) und dem Promotormodul (b).

Unter der Komponente (a), der Aktivierungssequenz, wird ein Teilabschnitt eines Gens verstanden, an den regulatorische Proteine, die sogenannten Transkriptionsfaktoren, binden können, welche in ihrer Gesamtheit die Transkription des stromabwärts gelegenen Strukturgens aktivieren.

Als "stromabwärts"-Sequenzen werden diejenigen Bereiche bezeichnet, die in Richtung der Transkription liegen, wohingegen Sequenzen, die in der entgegengesetzten Richtung angeordnet sind, als "stromaufwärts"-Sequenzen bezeichnet werden.

An das erfindungsgemäß verwendete, stromabwärts der Aktivierungssequenz gelegene Promotormodul binden sowohl Transkriptionsfaktoren der E2F-Familie als auch Transkriptionsfaktoren der CHF-Familie.

Zu der Familie der E2F-Moleküle gehören beispielsweise die Moleküle E2F1, E2F2, E2F3, E2F4 und E2F5, gegebenenfalls im Komplex mit DP1, DP2 oder DP3. Zu den E2F-Molekülen gehören aktivierende dimere Komplexe, wie beispielsweise E2F1-DP1 und trimere E2F-Komplexe, wie beispielsweise E2F1-DP1-pRb [Bandara et al., Nature, 252, 249 (1991); Chellappan et al., Cell 65, 1053 (1991); Chittenden et al., Cell 65, 1073 (1991); Helin et al., Cell 70, 337 (1992); Kaelin et al., Cell 70, 351 (1992)]. Die Komplexe E2F4-DP1 und E2F4-DP1-p107 wurden beispielsweise beschrieben von Beijersbergen et al., Genes Dev. 8, 2680 (1994) und Ginsberg et al., Genes Dev. 8, 2665 (1994). Weiterhin wären zu erwähnen entsprechende Komplexe enthaltend E2F2, E2F3, E2F5, DP2 und DP3.

Den Transkriptionsfaktoren, zu denen die E2F-Moleküle gehören, gemeinsam ist die Eigenschaft, daß eine Beziehung zwischen der Expression von Genen und dem Zustand, in dem sich die Zelle befindet, besteht. In ruhenden Zellen ist die Transkription der Gene sehr gering, wohingegen die Transkription in sich teilenden Zellen erhöht ist. Im Experiment mit kultivierten Zellen kann die Stimulierung der Expression durch Zugabe von Serum, bevorzugt fötales Kälberserum, zu ruhenden Zellen erreicht werden.

Zellen vermehren sich durch Zellteilung, wobei das Genom der Zelle vor der Mitose repliziert wird. Sich nicht teilende, ruhende Zellen befinden sich in einer als G0 bezeichneten Phase oder sind in ihrer Zellstatusprogression durch die G1-Phase inhibiert. Wenn die Zellteilung beginnt, werden die Zellen aus der G0-Phase bzw. dem G1-Block entlassen. Die G1-Phase ist eine üblicherweise verhältnismäßig lang andauernde Phase. In der G0- und G1-Phase befindet sich der DNA-Gehalt der Zelle im diploiden Zustand. An die G1-Phase schließt sich die S-Phase an, in der die DNA-Synthese stattfindet und in der das Genom dupliziert wird. Hieran schließt sich die zweite G-Phase, G2 an, in der die Zelle im tetraploiden Zustand ist. Daraufhin findet die Mitose statt und die Zelle kommt in einen G1- oder G0-Zustand.

Das B-myb Gen ist an der Zellteilung beteiligt. Die Transkription des B-myb Genes steigt (z.B. in Mäusefibroblasten) in der späten G1-Phase des Zellzyklus an und ist am stärksten ausgeprägt in der S-Phase (Lam et al., EMBO J. 12, 2705 (1993)).

Die Nukleotidsequenz CTTGGCGGGAG ist ein Bestandteil der Promotorsequenz des B-myb-Genes und an der Repression des Gens in der G0- und frühen G1-Phase beteiligt (Lam et al., EMBO J. 12, 2705 (1993)). TTTCGCGC ist ein Bestandteil des E2a Promotors des Adenovirus und repräsentiert eine Zone der aktivierenden E2F-Bindestellen (Mudryj et al., EMBO J. 9, 2179 (1990)).

Im Rahmen der vorliegenden Erfindung wurde nun gefunden, daß ein bestimmter Bereich aus der Promotorsequenz des B-myb-Genes die Expression eines Genes, das Teil eines erfindungsgemäßen Nukleinsäurekonstruktes ist, steuern kann. Diese Nukleotidsequenz, die erfindungsgemäß als Promotormodul (b) bezeichnet wird, ist wesentlich an der Regulation, insbesondere Repression des stromabwärts gelegenen Gens in der G0- und frühen G1-Phase beteiligt.

Das erfindungsgemäß verwendete Promotormodul (b) weist eine Nukleotidsequenz auf, an welche die Transkriptionsfaktoren der E2F-Familie binden können. Hierbei handelt es sich um folgende Sequenz:
CTTGGCGGG Seq.-ID 1
oder um analoge Varianten dieser Sequenz, wie beispielsweise
TTTCGCGCC Seq.-ID 2
TTTCGCGGC Seq.-ID 3.

Weiterhin weist das erfindungsgemäße Promotormodul (b) eine von der E2F-Stelle am 3'-Ende stromabwärts gelegene Sequenz auf, an die ein Molekül der CHF-Familie binden kann. Diese Nukleotidsequenz, die für die Repression ebenfalls notwendig ist, kooperiert mit der E2F-Bindestelle bei der Repression und umfaßt die Sequenz
TAGGAAA Seq.-ID 4
oder um analoge Varianten dieser Sequenz.

Im B-myb Gen liegen die Sequenz 1 und Sequenz 4 des erfindungsgemäßen Promotormoduls in folgender Zuordnung vor
CTTGGCGGGAGATAGGAAAGT Seq.-ID 5.

In besonders bevorzugter Ausführungsform beinhaltet das Promotormodul diese Sequenz.

Im Rahmen der vorliegenden Erfindung wurde nun festgestellt, daß dieses Promotormodul eine hochaffine E2F-Bindestelle aufweist und, daß die Bindung von E2F-Molekülen an diese Nukleotidsequenz (insbesondere CTTGGCGGG) in vivo, also in der lebenden Zelle, überraschenderweise (und im Gegensatz zu den von Lam et al., EMBO-J. 12, 2705 (1993) biochemischen (in vitro) Bindungsdaten) auf die G0- und frühe G1-Phase beschränkt und in der S, G2 und M Phase, (d.h. bei stärkster Transkription des B-myb Genes) nicht nachweisbar ist.

Weiterhin war nicht vorherzusehen, daß für die wirkungsvolle Repression eine weitere, von der E2F Bindestelle stromabwärts gelegene Bindungsregion für Proteine aus der CHF-Familie (bevorzugt TAGGAAA) erforderlich ist.

Da zum Zeitpunkt der Transkription des B-myb-Genes keine Bindung des E2F-Proteins an die Nukleotidsequenz CTTGGCGGGAG festgestellt wird, spielen die in der Zelle vorhandenen aktivierenden E2F-Dimere bei der Zellzyklusregulation des B-myb-Gens keine erkennbare Rolle. Somit ist die E2F-vermittelte Repression der ausschlaggebende Mechanismus bei der Regulation von B-myb.

Die Anordnung der erfindungsgemäßen Nukleinsäurekonstrukte kann in schematischer Weise dargestellt werden wie folgt:

5'

Aktivatorsequenz
(bevorzugt zell-
spezifisch oder
virusspezifisch)

zellzyklusreguliertes
Promotormodul,
enthaltend die Binde-
sequenz für Trans-
kriptionsfaktoren der
E2F- und CHF-Familie

Strukturgen für
einen Wirkstoff

3'

Die Aktivatorsequenz und das Strukturgen für den Wirkstoff der erfindungsgemäßen Nukleinsäurekonstrukte werden je nach Verwendungszweck ausgewählt. Bevorzugt sind folgende Anwendungsmöglichkeiten:

### 1) Therapie von Tumoren und chronischen Entzündungen über die Inhibition des proliferierenden Endothels

### 1.1.a) Aktivatorsequenzen aktiviert in Endothelzellen

Im Sinne dieser Erfindung zählen zu den bevorzugten Aktivatorsequenzen solche genregulatorische Sequenzen bzw. Elemente aus Promotoren für Gene, die für besonders in Endothelzellen (oder aber in Zellen in unmittelbarer Nachbarschaff mit proliferierenden Endothelzellen) nachweisbare Proteine kodieren.

Einige dieser Proteine sind von Burrows et al. (Pharmac. Therp. 64, 155 (1994)) und Plate et al. (Brain Pathol. 4, 207 (1994)) beschrieben worden. Im besonderen zählen zu diesen Endothelzell-spezifischen Proteinen beispielsweise:
- Hirn-spezifischer, endothelialer Glucose-1-Transporter Die Promotorsequenz wurde von Murakami et al. (J. Biol. Chem. 267, 9300 (1992)) beschrieben.
- Endoglin
   Die Promotorsequenz wurde von Bellon et al. (Eur. J. Immunol. 23, 2340 (1993)) und Ge et al. (Gene 138, 201 (1994)) beschrieben.
- VEGF-Rezeptoren
   Zwei Rezeptoren werden unterschieden (Plate et al., Int. J. Cancer 59, 520 (1994)):
   - VEGF-Rezeptor-1 (flt-1)
      (de Vries et al., Science 255, 989 (1992)) und der
   - VEGF-Rezeptor-2 (flk-1, KDR)
      (Terman et al., BBRC 187, 1579 (1992)).

Beide Rezeptoren sind fast ausschließlich auf endothelialen Zellen (Senger et al., Cancer Metast. Rev. 12, 303 (1993)) anzutreffen.
- andere endothelspezifische Rezeptortyrosinkinasen
   - til-1 oder til-2
      (Partanen et al., Mol. Cell. Biol. 12, 1698 (1992), Schnürch and Risau, Development 119, 957 (1993), Dumont et al., Oncogene 7, 1471 (1992))
   - B61-Rezeptor (Eck-Rezeptor)
      (Bartley et al., Nature 368, 558 (1994), Pandey et al., Science 268, 567 (1995), van der Geer et al., Ann. Rev. Cell. Biol. 10, 251 (1994))
- B61
   Das B61-Molekül stellt den Liganden dar für den B61-Rezeptor.
   (Holzman et al., J. Am. Soc. Nephrol. 4, 466 (1993), Bartley et al., Nature 368, 558 (1994))
- Endothelin, im speziellen
   - Endothelin B
      Die Promotorsequenz wurde von Benatti et al., J. Clin. Invest. 91, 1149 (1993) beschrieben.
   - Endothelin-1
      Die Promotorsequenz wurde von Wilson et al., Mol. Cell. Biol. 10, 4654 (1990) beschrieben.
- Endothelin-Rezeptoren, insbesondere der Endothelin-B
   Rezeptor
   (Webb et al., Mol. Pharmacol. 47, 730 (1995), Haendler et al., J. Cardiovasc. Pharm. 20, 1 (1992)).
- Mannose-6-Phosphat-Rezeptoren Die Promotorsequenzen sind von Ludwig et al. (Gene 142, 311 (1994)), Oshima et al. (J. Biol. Chem. 263, 2553 (1988)) und Pohlmann et al. (PNAS USA 84, 5575 (1987)) beschrieben worden.
- von Willebrand Faktor
   Die Promotorsequenz wurde von Jahroudi und Lynch, Mol. Cell. Biol. 14, 999 (1994), Ferreira et al., Biochem. J. 293, 641 (1993) und Aird et al., PNAS USA 92, 4567 (1995)) beschrieben.
- IL-1α, IL-1β
   Die Promotorsequenzen wurden von Hangen et al., Mol. Carcinog. 2, 68 (1986), Turner et al., J. Immunol. 143, 3556 (1989), Fenton et al., J. Immunol. 138, 3972 (1987), Bensi et al., Cell Growth Diff. 1, 491 (1990), Hiscott et al., Mol. Cell. Biol. 13, 6231 (1993) und Mori et al., Blood 84, 1688 (1994) beschrieben.
- IL-1-Rezeptor
   Die Promotorsequenz wurde von Ye et al., PNAS USA 90, 2295 (1993) beschrieben.
- Vascular Cell Adhesion Molecule (VCAM-1)
   Die Promotorsequenz des VCAM-1 wurde beschrieben von Neish et al., Mol. Cell. Biol. 15, 2558 (1995), Ahmad et al., J. Biol. Chem. 270, 8976 (1995), Neish et al., J. Exp. Med. 176, 1583 (1992), Iademarco et al., J. Biol. Chem. 267, 16323 (1992) und Cybulsky et al., PNAS USA 88, 7859 (1991).
- synthetische Aktivatorsequenz
   Als Alternative zu natürlichen endothelspezifischen Promotoren lassen sich auch synthetische Aktivatorsequenzen verwenden, die aus oligomerisierten Bindestellen für Transkriptionsfaktoren, die preferentiell oder selektiv in Endothelzellen aktiv sind, bestehen. Ein Beispiel hierfür ist der Transkriptionsfaktor GATA-2, dessen Bindestelle im Endothelin-1 Gen 5'-TTATCT-3' ist (Lee et al., Biol. Chem. 266, 16188 (1991), Dorfmann et al., J. Biol. Chem. 267, 1279 (1992) und Wilson et al., Mol. Cell. Biol. 10, 4854 (1990)).

### 1.1.b) Aktivatorsequenzen aktiviert in Zellen in Nachbarschaft aktivierter Endothelzellen

Bei proliferierenden Endothelien werden durch geöffnete "tight junctions" Nachbarzellen für Makromoleküle vom Blut aus zugänglich. Durch die funktionellen und anatomischen Wechselbeziehungen sind die Nachbarzellen von aktivierten Endothelzellen Zielzellen im Sinne dieser Erfindung.
- VEGF
   Die genregulatorischen Sequenzen für das VEGF-Gen sind
   - die Promotorsequenz des VEGF-Gens (5' flankierende Region) (Michenko et al., Cell. Mol. Biol. Res. 40, 35 (1994), Tischer et al., J. Biol. Chem. 266, 11947 (1991)) oder
   - die Enhancersequenz des VEGF-Gens (3' flankierende Region) (Michenko et al., Cell. Mol. Biol. Res. 40, 35 (1994)) oder
   - das c-Src-Gen
      (Mukhopadhyay et al., Nature 375, 577 (1995), Bonham et al., Oncogene 8, 1973 (1993), Parker et al., Mol. Cell. Biol. 5, 831 (1985), Anderson et al., Mol. Cell. Biol. 5, 1122 (1985)) oder
   - das V-Src-Gen
      (Mukhodpadhyay et al., Nature 375, 577 (1995), Anderson et al., Mol. Cell. Biol. 5, 1122 (1985), Gibbs et al., J. Virol. 53, 19 (1985))
- Steroid-Hormonrezeptoren und deren Promotorelemente (Truss and Beato, Endocr. Rev. 14, 459 (1993)), insbesondere der
   - Maus-Mammatumor-Virus-Promotor
      Die cDNA-Sequenz der Promotorregion der long terminal repeat region des MMTV wurde von Chalepakis et al., Cell 53, 371 (1988) und Truss und Beato (Endocr. Rev. 14, 459 (1993)) beschrieben.

### 1.2.) Strukturgene für antitumorale (oder antientzündliche) Substanzen

### 1.2.a) Inhibitoren der Proliferation

Als antitumorale oder antientzündliche Substanz im Sinne dieser Erfindung ist die DNA-Sequenz eines Proteins zu verstehen, welches die Proliferation von Endothelzellen inhibiert. Hierzu gehören beispielsweise die DNA-Sequenzen für:
- das Retinoblastomprotein (pRb/p110) oder für seine Analoga p107 und p120
- das p53 Protein
- das p21 (WAF-1) Protein
- das p16 Protein
- weitere CdK-Inhibitoren
- das GADD45 Protein
- das bak Protein

Um eine schnelle intrazelluläre Inaktivierung dieser Zellzyklusinhibitoren zu verhindern, sind bevorzugt solche Gene zu verwenden, welche Mutationen für die Inaktivierungsstellen der exprimierten Proteine aufweisen, ohne daß diese hierdurch in ihrer Funktion beeinträchtigt werden.

Das Retinoblastomprotein (pRb) und die Verwandten p107 und p130 Proteine werden durch Phosphorylierung inaktiviert. Bevorzugt wird somit eine pRb/p110-, p107 - oder p130 cDNA-Sequenz verwendet, die derartig punktmutiert ist, daß die Phosphorylierungsstellen des kodierten Proteins gegen nicht phosphorylierbare Aminosäuren ausgetauscht sind.

### 1.2.b) Angiogeneseinhibitoren

Als antitumorale bzw. antientzündliche Substanz ist desweiteren die DNA-Sequenz für ein Protein zu verstehen, welches die Angiogenese inhibiert. Zu diesen Proteinen zählt beispielsweise:
- Tissuefactor (TF) und gerinnungsaktive Fragmente von ihm
- Plasminogenaktivatorinhibitor-1 (PAI-1)
- PAI-2
- PAI-3
- Angiostatin
- Interferone
- IFNα
- IFNβ
- IFNγ
- Platelet factor 4
- IL-12
- TIMP-1
- TIMP-2
- TIMP-3
- Leukemia Inhibitory Factor (LIF)

### 1.2.c) zytostatische und zytotoxische Proteine

Als antitumorale bzw. antientzündliche Substanz ist jedoch auch eine DNA-Sequenz für ein Protein zu verstehen, welches direkt oder indirekt eine zytostatische Wirkung auf Tumoren aufweist. Hierzu zählen im besonderen
- Antikörper und Antikörperspaltprodukte
- Perforin
- Granzym
- IL-2
- IL-4
- IL-12
- Interferone, wie beispielsweise
   * IFN-α
   * IFN-β
   * IFN-γ
- TNF
   * TNF-α
   * TNF-β
- Oncostatin M
- Magainin und Magininderivate (Cruciani et al. PNAS 88, 3792 (1991); Peck-Miller et al.; Cancer Chemoth. Pharmac. 32, 109, (1993))
- Sphingomyelinase (Jarvis et al. PNAS-USA 91, 73 (1994))

### 1.2.d) Induktoren von Entzündungen

Als antitumorale Substanz ist des weiteren die DNA-Sequenz für ein Protein zu verstehen, welches ggf. zusätzlich zur antitumoralen Wirkung Entzündungen stimuliert und hierdurch zur Elimination von Tumorzellen beiträgt. Hierzu zählen im besonderen beispielsweise:
- RANTES (MCP-2)
- monocyte chemotactic and activating factor (MCAF)
- IL-8
- macrophage inflammatory protein-1 (MIP-1a, -β)
- neutrophil activating protein-2 (NAP-2)
- IL-3
- IL-5
- human leukemia inhibitory factor (LIF)
- IL-7
- IL-11
- IL-13
- GM-CSF
- G-CSF
- M-CSF
- Bakterielle Proteine, welche Komplement aktivieren
- Cobra Venom Faktor (CVF) und Teilsequenzen vom CVF

Als Wirksubstanz in Sinne der Erfindung können auch DNA-Sequenzen von Fusionsproteinen zwischen den aufgeführten Cytokinen zum einen und dem Fc-Teil des menschlichen Immunglobulins zum anderen Verwendung finden. Derartige DNA-Sequenzen und ihre Herstellung wurden in der EP 0464 633 A1 beschrieben.

### 1.2.e) Enzyme für die Aktivierung von Vorstufen von Zytostatika

Als antitumorale bzw. antientzündliche Substanz ist jedoch auch die DNA-Sequenz für ein Enzym zu verstehen, welches in der Lage ist, Vorstufen eines antitumoralen Wirkstoffes in einen antitumoralen Wirkstoff zu überführen.

Derartige Enzyme, welche inaktive Vorsubstanzen (Prodrugs) in aktive Zytostatika (Drugs) spalten und die jeweils zugehörigen Prodrugs und Drugs sind bereits von Deonarain et al. Br. J. Cancer 70, 786 (1994), von Mullen, Pharmac. Ther. 63, 199 (1994) und Harris et al., Gene Ther. 1, 170 (1994) übersichtlich beschrieben worden.

Beispielsweise ist die DNA-Sequenz eines der folgenden Enzyme zu verwenden:
- Herpes Simplex Virus Thymidinkinase
   (Garapin et al., PNAS USA 76, 3755 (1979), Vile et al., Cancer Res. 53, 3860 (1993), Wagner et al., PNAS USA 78, 1441 (1981), Moelten et al., Cancer Res. 46, 5276 (1986), J. Natl. Cancer Inst. 82, 297 (1990))
- Varizella Zosier Virus Thymidinkinase (Huber et al., PNAS USA 88, 8039 (1991), Snoeck, Int. J. Antimicrob. Agents 4, 211 (1994))
- bakterielle Nitroreduktase
   (Michael et al., FEMS Microbiol. Letters 124, 195 (1994), Bryant et al., J. Biol. Chem. 266, 4126 (1991), Watanabe et al., Nucleic Acids Res. 18, 1059 (1990))
- bakterielle β-Glucuronidase
   (Jefferson et al., PNAS USA 83, 8447 (1986))
- pflanzliche β-Glucuronidase aus Secale cereale
   (Schulz et al., Phytochemistry 26, 933 (1987))
- humane β-Glucuronidase
   (Bosslet et al., Br. J. Cancer 65, 234 (1992), Oshima et al., PNAS USA 84, 685 (1987))
- humane Carboxy peptidase (CB) z.B.
   * CB-A der Mastzelle
      (Reynolds et al., J. Clin. Invest. 89, 273 (1992))
   * CB-B des Pankreas
      (Yamamoto et al., J. Biol. Chem. 267, 2575 (1992), Catasus et al., J. Biol. Chem. 270, 6651 (1995))
   * bakterielle Carboxy peptidase
      (Hamilton et al., J. Bacteriol. 174, 1626 (1992), Osterman et al., J. Protein Chem. 11, 561 (1992))
- bakierielle β-Laktamase
   (Rodrigues et al., Cancer Res. 55, 63 (1995), Hussain et al., J. Bacteriol. 164, 223 (1985), Coque et al., Embo J. 12, 631 (1993))
- bakterielle Cytosine deaminase
   (Mullen et al., PNAS USA 89, 33 (1992), Austin et al., Mol. Pharmac. 43, 380 (1993), Danielson et al., Mol. Microbiol. 6, 1335 (1992))
- humane Catalase bzw. Peroxidase
   (Ezurum et al., Nucl. Acids Res. 21, 1607 (1993))
- Phosphatase, im besonderen
   * humane alkalische Phosphatase
      (Gum et al., Cancer Res. 50, 1085 (1990))
   * humane saure Prostataphosphatase
      (Sharieff et al., Am. J. Hum. Gen. 49, 412 (1991), Song et al., Gene 129, 291 (1993), Tailor et al., Nucl. Acids Res. 18, 4928 (1990))
   * Typ 5 saure Phosphatase
      (Gene 130, 201 (1993))
- Oxidase, im besonderen
   * humane Lysyloxidase
      (Kimi et al., J. Biol. Chem. 270, 7176 (1995))
   * humane saure D-aminooxidase
      (Fukui et al., J. Biol. Chem. 267, 18631 (1992))
- Peroxidase, im besonderen
   * humane Glutathion Peroxidase
      (Chada et al., Genomcis 6, 268 (1990), Ishida et al., Nucl. Acids Res. 15, 10051 (1987))
   * humane Eosinophilen Peroxidase
      (Ten et al., J. Exp. Med. 169, 1757 (1989), Sahamaki et al., J. Biol. Chem. 264, 16828 (1989))
   * humane Schilddrüsen Peroxidase
      (Kimura, PNAS USA 84, 5555 (1987)).

Zur Erleichterung der Sekretion der aufgeführten Enzyme kann die jeweils in der DNA-Sequenz enthaltende homologe Signalsequenz ersetzt werden durch eine heterologe, die extrazelluläre Ausschleusung verbessernde Signalsequenz.

So kann beispielsweise die Signalsequenz der β-Glucuronidase (DNA Position 〈 27 bis 93; Oshima et al., PNAS 84, 685 (1987)) ersetzt werden durch die Signalsequenz für das Immunglobulin (DNA Position 〈 63 bis 〉 107; Riechmann et al., Nature 332, 323 (1988)).

Des weiteren sind bevorzugt DNAs solcher Enzyme zu wählen, welche durch Punktmutationen in einem geringeren Maße in Lysosomen gespeichert und vermehrt sekretiert werden.

### 1.3. Kombination von mehreren antitumoralen oder antientzündlichen Substanzen

Gegenstand der Erfindung sind desweiteren Nukleinsäurekonstrukte, in welchen eine Kombination der DNA-Sequenzen von mehreren gleichen antitumoralen oder antientzündlichen Substanzen (A,A) oder von unterschiedlichen antitumoralen Substanzen (A,B) vorliegt. Zur Expression von zwei DNA-Sequenzen wird vorzugsweise die cDNA einer "internal ribosome entry site" (IRES) als regulatorisches Element zwischengeschaltet.

Aktivator-
sequenz
zellzyklus-
reguliertes
Promotor-
modul
antitumorale
Substanz A
internal
ribosome
entry site
antitumorale
Substanz
A oder B

Derartige IRES wurden beispielsweise von Mountford und Smith TIG 11, 179 (1995), Kaufman et al., Nucl. Acids Res. 19, 4485 (1991), Morgan et al., Nucl. Acids Res. 20, 1293 (1992, Dirks et al., Gene 128, 247 (1993), Pelletier und Sonenberg, Nature 334, 320 (1988) und Sugitomo et al., BioTechn. 12, 694 (1994) beschrieben.

So kann die cDNA der IRES-Sequenz des Poliovirus (Position 〈 140 bis 〉 630 des 5' UTR; Pelletier und Sonenberg, Nature 334, 320 (1988)) zur Verknüpfung der DNA der antientzündlichen Substanz A (am 3' Ende) und der DNA der antientzündlichen Substanz B (am 5' Terminus) verwendet werden.

Ein derartiger Wirkstoff weist je nach Kombination additive (A+A, A+B1) oder synergistische Wirkung im Sinne der Erfindung auf.

### 2) Wirkstoff zur Behebung der mangelhaften Bildung von Zellen des Blutes

### 2.1.) Auswahl der Aktivatorsequenz für blutbildende Zellen

Im Sinne der vorliegenden Erfindung wird als Aktivatorsequenz bevorzugt eine genregulatorische Sequenz bzw. ein Element aus einem Gen verwendet, welches ein Protein kodiert, welches besonders stark oder selektiv in Zellen der Blutbildung exprimiert ist. Zu solchen genregulatorischen Sequenzen gehören Promotorsequenzen für Gene eines Cytokins oder seines Rezeptors, dessen Expression in den unreifen blutbildenden Zellen (oder in benachbarten Zellen, wie beispielsweise dem Stroma), dem nachfolgenden, auf die blutbildenden Zellen einwirkenden und als Wirksubstanz gewünschten Cytokin vorgeschaltet ist. Beispielsweise sind derartige auf unreife blutbildende Zellen einwirkende Cytokine wie:
- Stem Cell Factor
- IL-1
- IL-3
- IL-6
   GM-CSF

### 2.2.) Auswahl der Strukturgene für Wirksubstanzen für blutbildende Zellen

Als Wirksubstanz im Sinne der Erfindung ist eine DNA-Sequenz zu verstehen, deren exprimiertes Protein die Proliferation und/oder Differenzierung von Blutzellen bewirkt.

### 3) Wirkstoff zur Therapie von Autoimmunerkrankungen, Allergien, Entzündungen und zur Verhütung von Organabstoßungen

### 3.1.) Auswahl der Aktivatorsequenz für Autoimmunerkrankungen u.a.

Als Aktivatorsequenzen sind die Promotorsequenzen der Gene für solche Proteine zu verwenden, welche bei der Immunreaktion in Makrophagen und/oder in Lymphozyten verstärkt gebildet werden. Derartige Proteine sind beispielsweise
- IL-1
- IL-1-β
- IL-1 -Rezeptor
- IL-2
- IL-2-Rezeptor
- IL-3
- IL-3-Rezeptor
- IFN-γ
- IL-4
- IL-4-Rezeptor
- IL-5
- IL-6
- LIF
- IL-7
- IL-10
- IL-11
- IL-12
- IL-13
- IM-CSF
- GM-CSF-Rezeptor
- Integrin beta 2 proteins

### 3.2.) Auswahl der Gene für Wirksubstanzen für Autoimmunerkrankungen u.a.

Die Wirksubstanz im Sinne der Erfindung ist die DNA-Sequenz für ein Cytokin, ein Chemokin, einen Wachstumsfaktor oder einer ihrer Inhibitoren, einen Antikörper, ein Antikörperfragment, einen Enzyminhibitor oder ein Enzym. Die Auswahl der Wirksubstanz richtet sich nach der zu behandelnden Grunderkrankung und der gewählten Promotorsequenz.

### 4) Wirkstoff zur Behandlung der Arthritis

### 4.1.) Auswahl der Aktivatorsequenz für Arthritis

Als Aktivatorsequenz ist eine Nukleotidsequenz (Promotor- oder Enhancersequenz) zu verstehen, mit der Transkriptionsfaktoren gebildet oder aktiv in Synovialzellen und Entzündungszellen interagieren. Im Sinne dieser Erfindung zählen zu den bevorzugten Promotorsequenzen genregulatorische Sequenzen bzw. Elemente aus Genen, die für besonders in Synovialzellen und Entzündungszellen exprimierte Proteine kodieren.

### 4.2.) Auswahl der Strukturgene für Wirksubstanzen für Arthritis

Als Wirksubstanz im Sinne der Erfindung ist eine DNA-Sequenz zu verstehen deren exprimiertes Protein die Entzündung beispielsweise im Gelenk direkt oder indirekt hemmt und/oder die Rekonstitution von extrazellulärer Matrix (Knorpel, Bindegewebe) im Gelenk fördert.

### 5) Herstellung eines Wirkstoffes gegen Infektionserreger

Der Wirkstoff kann in zwei grundsätzlich unterschiedlichen Formen hergestellt werden
- für die Therapie von Virusinfektionen und Parasiteninvasionen oder aber
- für die Prophylaxe von Infektionserkrankungen durch Viren, Bakterien oder Parasiten.

Zur Prophylaxe von Infektionserkrankungen dienen Impfstoffe. Die Möglichkeiten, auf konventionellem Wege wirkungsvolle Impfstoffe herzustellen, sind jedoch beschränkt (Brown, Int.J. Technol. Assessm. Health Care 10, 161 (1994), Ellis, Adv. Exp. Med. Biol. 327, 263 (1992), Arnon et al., FASEB J. 6, 3265 (1992)).

Demzufolge wurde die Technologie der DNA-Vakzine ent▼wickelt. Diese DNA-Vakzinen werfen jedoch Fragen zur Sicherheit und zu Nebenwirkungen auf (Fynan et al., Int. J. Immunopharm. 17, 79 (1995), Donnelly et al., Immunol. 2, 20 (1994)).

Wirkstoffe zur Prophylaxe von Infektionserkrankungen im Sinne dieser Erfindung zeichnen sich wegen ihrer Zellspezifität und Zellzyklusregulation durch ein hohes Maß an Sicherheit aus.

### 5.1.) Auswahl der Aktivatorsequenz

### a) zur Therapie von Infektionserkrankungen

Als Aktivatorsequenz sind Promotorsequenzen von Zellgenen auszuwählen, deren Aktivität besonders durch Infektionen mit Bakterien oder Parasiten verändert wird oder es sind Promotorsequenzen solcher Viren auszuwählen, die die von ihnen infizierten Zellen transformieren und zur Proliferation anregen.

Zu diesen Viren gehören beispielsweise HBV, HCV, HSV, HPV, HIV, EBV und HTLV.

### 5.2.) Auswahl der Sirukturgene für Wirksubstanzen

### a) zur Therapie von Infektionserkrankungen

Als Wirksubstanz ist die DNA eines Proteins auszuwählen, welches zytostatische, zytotoxische, antibakterielle oder antivirale Wirkungen aufweist. Beispiele für zytotoxische oder zytostatische Proteine wurden schon oben aufgeführt. Als Beispiel für antibakterielle oder antivirale Proteine sind Antikörper oder Antikörperfragmente zu nennen. Bei der Wahl eines Enzyms ist nachfolgend die durch dieses Enzym spaltbare Vorstufe einer antibakteriellen, antiviralen zytotoxischen oder antiparasitären Substanz zu verabreichen.

Wirksubstanzen für antivirale Proteine im Sinne dieser Erfindung sind des weiteren antiviral wirksame Cytokine und Wachstumsfaktoren. Hierzu zählen beispielsweise die DNA-Sequenzen für folgende Wirksubstanzen:
- IFN-α
- IFN-β
- IFN-γ
- TNF-β
- TNF-α
- IL-1
- TGF-β

Als Wirksubstanz im Sinne der Erfindung können jedoch auch DNA-Sequenzen von Fusionsproteinen zwischen den aufgeführten Cytokinen, Wachstumsfaktoren oder dem extrazellulären Teil der Rezeptoren zum einen und dem Fc-Teil des menschlichen Immunglobulins zum anderen Verwendung finden. Derartige DNA-Sequenzen und ihre Herstellung wurden beispielsweise in der EPA 0464 633 A1 beschrieben.

### b) zur Prophylaxe von Infektionserkrankungen

Als Wirksubstanz ist die DNA entweder eines Antikörpers oder eines Antikörperfragmentes spezifisch für den Infektionserreger oder die DNA eines vom Infektionserreger gebildeten Proteins auszuwählen, welches durch eine Immunreaktion, d.h. durch Antikörperbindung und/oder durch zytotoxische T-Lymphozyten zur Neutralisierung und/oder zur Abtötung des Erregers führt. Derartige Neutralisationsantigene werden als Impfantigene bereits angewandt (siehe Übersicht bei Ellis, Adv. Exp. Med. Biol. 327, 263 (1992)).

### 6) Wirkstoff zur Behandlung von Leukämien und Tumoren

### 6.1.) Auswahl der Aktivatorsequenz für Leukämien und Tumoren

Als Aktivatorsequenz ist eine Nukleotidsequenz (Promotor- oder Enhancersequenz) vorgesehen, mit der Transkriptionsfaktoren, gebildet oder aktiv in Leukämiezellen oder Tumorzellen, interagieren.

Im Sinne dieser Erfindung zählen zu den bevorzugten Aktivatorsequenzen jedoch genregulatorische Sequenzen bzw. Elemente aus Genen, die besonders in Tumorzellen oder Leukämiezellen gebildete Proteine kodieren.

### 6.2.) Auswahl der Strukturgene für Wirksubstanzen für Leukämien und Tumorzellen

Als Wirksubstanz im Sinne der Erfindung ist eine DNA-Sequenz zu verstehen deren exprimiertes Protein die Proliferation von Zellen, insbesondere auch von Tumorzellen oder Leukämiezellen, inhibiert. Zu diesen Zellzyklusinhibitoren gehören beispielsweise die DNA-Sequenzen für inhibitorische zytostatische und ztyotoxische Proteine und Enzyme, wie sie bereits beschrieben wurden.

Als Zellzyklusinhibitor ist des weiteren eine DNA-Sequenz zu verstehen, welche ein Protein exprimiert, welches direkt oder indirekt eine zytostatische oder zytotoxische Wirkung auf Leukämien oder Tumoren aufweist.

### 7) Wirkstoff zur Inhibition der Proliferation glatter Muskelzellen bei Gefäßverschlüssen

### 7.1.) Auswahl der Aktivatorsequenz für glatte Muskelzellen

Als Aktivatorsequenzen im Sinne der Erfindung sind bevorzugt genregulatorische Sequenzen bzw. Elemente aus Genen zu verwenden, die, besonders in glatten Muskelzellen, gebildete Proteine kodieren.

### 7.2.) Auswahl der Strukturgene für Wirksubstanzen für glatte Muskelzellen

Als Wirksubstanz in Sinne der Erfindung ist eine DNA-Sequenz zu verstehen, deren exprimiertes Protein die Proliferation von glatten Muskelzellen inhibiert. Zu diesen Inhibitoren der Proliferation gehören die bereits erwähnten Proteine.

Als Wirksubstanz ist jedoch auch die DNA-Sequenz für ein Enzym zu verstehen, welches eine inaktive Vorstufe eines Zytostatikums in ein Zytostatikum umwandelt.

### 8) Wirkstoff zur Inhibition oder Aktivierung der Gerinnung

### 8.1.) Auswahl der Aktivatorsequenz zur Inhibition oder Aktivierung der Gerinnung

Als Aktivatorsequenzen im Sinne der Erfindung sind bevorzugt genregulatorische Sequenzen bzw. Elemente aus Genen zu verwenden, welche in glatten Muskelzellen, in aktivierten Endothelzellen, in aktivierten Makrophagen oder in aktivierten Lymphozyten nachweisbare Proteine kodieren.

### a) glatte Muskelzellen

Beispiele für Promotorsequenzen für Gene in glatten Muskelzellen sind bereits erwähnt.

### b) aktivierte Endothelzellen

Beispiele für Proteine, welche besonders in aktivierien Endothelzellen gebildet werden, sind von Burrows et al. (Pharmac. Therp. 64, 155 (1994)) beschrieben worden. Im besonderen zählen zu diesen in Endothelzellen verstärkt auftretenden Proteinen beispielsweise solche Proteine, wie sie und die Promotorsequenzen ihrer Gene bereits im oben aufgeführt wurden.

### c) aktivierie Makrophagen und/oder aktivierte Lymphozyten

Als Aktivatorsequenz im Sinne dieser Erfindung sind außerdem Promotorsequenzen der Gene für Proteine zu verstehen, welche bei der Immunreaktion in Makrophagen und/oder in Lymphozyten verstärkt gebildet werden.

### 8.2.) Auswahl der Strukturgene für Wirksubstanzen zur Inhibition oder Aktivierung der Gerinnung

Als Wirksubstanz im Sinne dieser Erfindung ist eine DNA▼Sequenz zu verwenden, welche ein Protein kodiert, welches direkt oder indirekt die Thrombozytenaggregation oder einen Blutgerinnungsfaktor inhibiert oder die Fibrinolyse stimuliert.

Eine derartige Wirksubstanz wird als Gerinnungshemmer bezeichnet. Als Gerinnungshemmer sind Gene für beispielsweise Plasminogenaktivatoren (PA), so der Gewebe-PA (tPA) oder der Urokinase ähnliche PA (uPA) oder Protein G, Antithrombin-III, C-1S-Inhibitor, µ1-Antitrypsin, der Tissue Facior Pathway Inhibitor (TFPI) oder Hirudin einzusetzen.

Als Wirksubstanz im Sinne dieser Erfindung ist jedoch auch eine DNA-Sequenz zu verwenden, welche ein Protein kodiert, welches die Blutgerinnung fördert. Beispiele für derartige Proteine sind beispielsweise Blutplasmaproteine wie Faktor VIII oder Faktor IX.

### 9) Wirkstoff zum Schutz vor CNS-Schäden

### 9.1) Aktivatorsequenzen für einen Wirkstoff zum Schutz vor CNS-Schäden

### 9.1.a) Aktivatorsequenzen, aktiviert in Endothelzellen

Im besonderen zählen hierzu die Promotorsequenzen für die Gene von Endothelzell-spezifischen Proteinen.

### 9.1.b) Aktivatorsequenzen, aktiviert in Gliazellen

Als bevorzugte Aktivatorsequenz ist desweiteren eine Nukleotidsequenz (Promotor- oder Enhancersequenz) zu verstehen, welche mit Transkriptionsfaktoren, im besonderen Maße gebildet oder aktiv in Gliazellen, interagieren.

### 9.2) Wahl der Strukturgene für neurospezifische Faktoren

Als neurospezifischer Faktor im Sinne der Erfindung ist eine DNA-Sequenz zu verstehen, welche für einen neuronalen Wachstumsfaktor kodiert.

Anhand der nachfolgenden Beispiele wird die vorliegende Erfindung näher erläutert.

### Beispiel 1

### Biochemische Untersuchungen

Die Wechselwirkung zwischen der Nukleotidsequenz CTTGCGGGAG des B-myb Promotors und den E2F-Komplexen wurde durch den "electrophoretic mobility shift assay" (EMSA) und durch "methylation protection footprinting" untersucht.

Der EMSA wurde durchgeführt wie von Barberis et al., Cell 50, 347 (1987) beschrieben.

Hierzu wurden folgende GST-Fusionsproteine in E. coli exprimiert und durch Affinitätschromatographie gereinigt:
- E2F1, Aminosäuren 87-1398 (Helin et al., Cell 70, 337 (1992) and Kaelin et al., Genes Dev. 8, 2665 (1994))
- E2F4, Gesamtprotein (Beijersbergen et al., Genes Dev. 8, 2680 (1994) and Ginsberg et al., Genes Dev. 8, 2665 (1994))
- pRb, Aminosäuren 300-928 (Bernards et al., Proc. Natl. Acad. Sci. USA 86, 6474 (1989))
- p107, Aminosäuren 744-2460 (Ewen et al., Cell 66, 1155 (1991).

Die gereinigten Proteine (etwa 100 ng) wurden inkubiert mit etwa 0,5 pmol einer ³²P-markierten Sonde für 30 min bei 22 °C in 15 µl eines Puffers der 50 mM Tris/HCl pH 8.0, 50 mM NaCl, 10% v/v Glycerol, 0.2 mM EDTA, 1 mM DTT und 67 µg poly[dA/dT]/µl enthielt. Die Sonden wurden markiert durch Einfügung der an den 5' überhängenden Enden von 4-7 Basen. Die Proben wurden in 4%igem, nicht denaturierendem Polyacrylamid-Gel in 0.5x TBE bei 4 °C und 10 V/cm aufgetrennt. Die Gele wurden exponiert, mit einem Röntgenstrahlen-empfindlichen Film und quantitativ analysiert mit Hilfe eines "Molecular Dynamics Phosphor-Imager".

Die folgenden Sonden wurden benutzt:
- B-myb [Lam et al., EMBO J. 12, 2705 (1993)],
   5'-GGCGCCGACGCACTTG GCGGGAGATAGGAAAGTGGTTCTGTG (Seq.-ID 6) (die E2F Bindungsstelle ist unterstrichen)
- mutiertes B-myb:
   5'-GGCGCCGACGCACTT GGCTGGAGATAGGAAAGT GGTTCTGTG (Seq.-ID 7)
- der E2a Promotor des Adenovirus:
   5'-gatcGACTAGT TTCGCGCCCTTTCTActag (Seq.-ID 8) (die kleinen Buchstaben stellen nicht relevante Sequenzen dar, die für die "fill-in labelling reactions" benutzt wurden)
- Kontrollsonde:
   5'-GATCCTCTCACCTGCTGCTAG. (Seq.-ID 9)

Das "methylation protection foot printing" wurde wie folgt durchgeführt:

Die B-myb kodierende Oligonukleotidsequenz wurde am Ende markiert, gereinigt und verbunden mit dem nicht kodierenden Strang. Die Bindungssreaktion wurde, wie für EMSA bereits beschrieben, durchgeführt. Zwei Mikroliter von 2% Dimethylsulfat (DMS) wurden zugefügt und die Methylierungsreaktion wurde 3 Minuten später gestoppt durch Zugabe von 2 µl von 60 mM β-Mercaptoethanol. Die Proben wurden in einem 4% Gel aufgetrennt und auf Ionenaustauschpapier übertragen. Die Banden wurden ausgeschnitten, gewaschen in TE-Puffer und eluiert bei 65 °C mit TE-Puffer enthaltend 1,5 M NaCl. Die eluierte DNA wurde mit Chloroform extrahiert, prezipitiert und in Wasser gelöst. Gleiche radioaktive Mengen der am Auftragungsort verbliebenen Bande (freie Probe) und der fortbewegten Bande (Komplex) wurden mit 10% Piperidin bei 95 °C für 30 Minuten gespalten. Die DNA wurde prezipitiert und auf ein 15% denaturierendes Acrylamid-Gel aufgetragen.

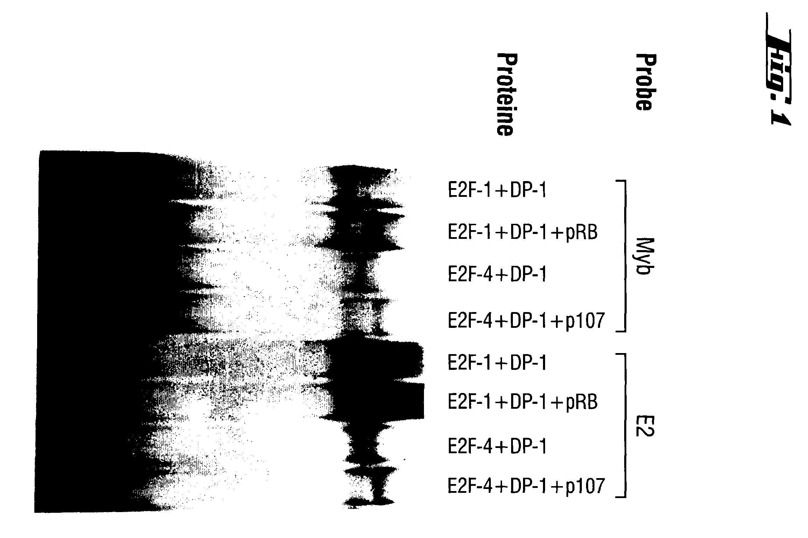

Wie in Abb. 1 gezeigt, binden E2F1-DP1, E2F1-DP1-pRb, E2F4-DP1 und
E2F4-DP1-p107 an die E2F Bindungsstelle des B-myb Promotors mit vergleichbarer Stärke und mit ähnlicher Affinität wie zu der hochaffinen
E2a-Promotorbindungsstelle des Adenovirus (Mudryj et al., EMBO J. 9, 2179 (1990)).

Die Quantifizierung der Bindung der E2F-Komplexe an die E2F-Bindungsstelle des B-myb Promotors wurde am EMSA Gel ermittelt mit Hilfe eines "Phosphor-Imager", der die Menge der gebundenen Probe ermittelte. Die folgenden Ergebnisse wurden erhalten (Anteil der gebundenen Probe):
- E2a-Promotor E2F Bindungsstelle: 1.0% (E2F1-DP1) bzw. 0.8% (E2F4-DP1)
- B-myb: 0.7% (E2F1-DP1) bzw. 2.0% (E2F1-DP1 bzw. 2.0% (E2F4-DP1)
- mutiertes B-myb: < 0.01%
- Negativkontrollprobe: < 0.002%.

E2F4 Komplexe wurden für eine Dimethylsulfat (DMS) "protection footprinting" Analyse ausgewählt, da das Protein p107 als das wesentliche Bindungsprotein in E2F Komplexen angesehen wird, die mit der Bindungsstruktur reagieren (Lam et al., EMBO J. 13, 871 (1994)) und E2F4 seinerseits das einzige bekannte Mitglied der E2F-Familie ist, welches mit p107 reagiert (Beijersbergen et al., Genes Dev. 8, 2680 (1994) and Ginsberg et al., Genes Dev. 8, 2665 (1994)).

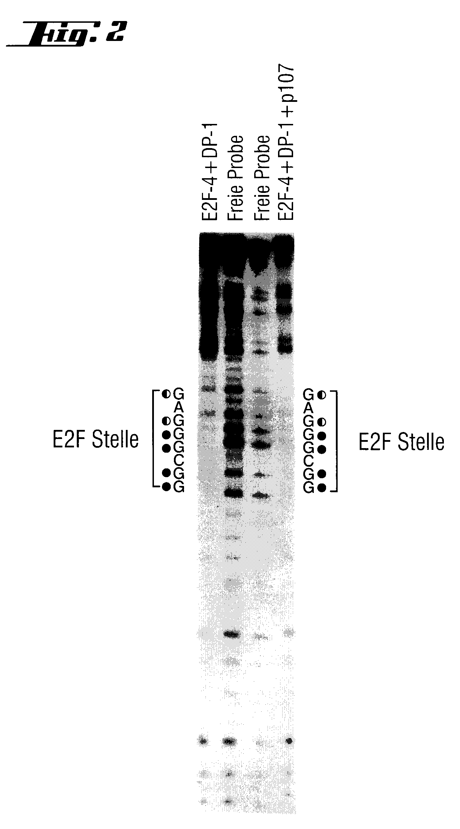

Wie in Fig. 2 dargestellt, waren die Nukleotide in der Region der E2F-Bindungssielle geschützt (CTTGGCGGGAG, geschützte Guanine sind unterstrichen, wobei die zwei am äußersten 3' Ende Iokalisierten Guanine stromabwärts der E2F-Kernbindungstelle nur einen Teilschutz zeigten). Die Ergebnisse zeigen des weiteren, daß keine Unterschiede nachweisbar waren zwischen den "footprints" erhalten mit E2F4-DP1 ("freies E2F") und dem E2F4-DP1-p107. Diese Beobachtung zeigt klar, daß E2F-Komplexe unabhängig vom Protein p107 an die DNA binden und daß diese DNA-Bindung durch "methylation protection footprinting" nachgewiesen werden kann. In Übereinstimmung hierzu steht der Befund, daß Assoziations- und Dissoziationsraten für E2F4-DP1 und E2F4-DP1-p107-Komplexe sehr ähnlich sind.

### Beispiel 2

### Untersuchungen an Zellen

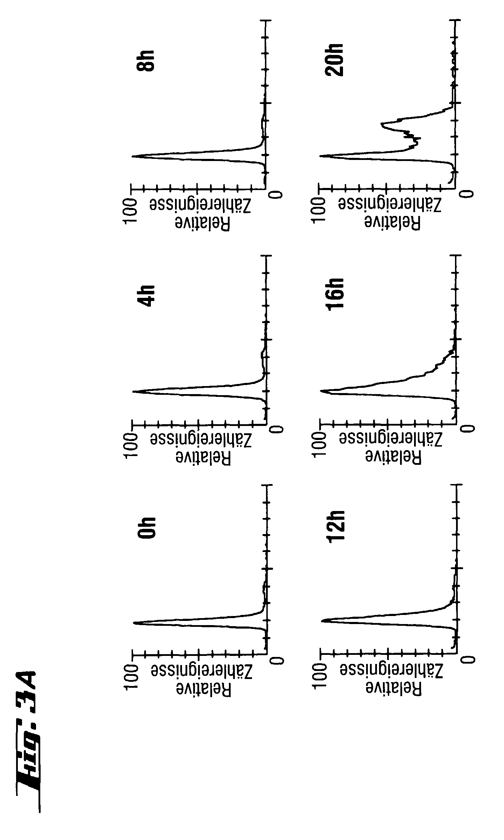

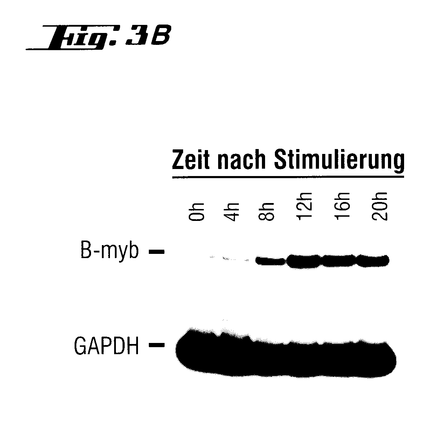

Um die Bindung von E2F an den B-myb-Promotor nicht nur biochemisch sondern auch an der lebenden Zelle nachzuweisen, wurden NIH3T3 Fibroblasten durch Serumentzug in G0 synchronisiert und nachfolgend mit 10 % FKS stimuliert. Zu unterschiedlichen Zeiten nach der Stimulierung wurde die Zellzyklusprogression (DNA-Gehalt), die B-myb mRNA-Expression und die Bindung von E2F-Komplexen an der E2F-Bindungsstelle (Schutz vor Methylierung) in vivo untersucht. Wie in Fig. 3A dargestellt, führt die Stimulation von ruhenden Zellen nach etwa 12 Stunden zum Eintritt in die S-Phase.

Wie bereits von Lam et al., EMBO J. 12, 2705 (1993) dargestellt, steigt die B-myb mRNA-Expression 8 Stunden nach der Simulation (d.h. in der späten G1-Phase) an und erreicht den größten Wert nach etwa 12 Stunden, d.h. zum Zeitpunkt des Eintretens in die S-Phase.

Um die Rolle der E2F-Bindungsstelle in der Zelle zu analysieren, wurde ein genomisches DMS "footprinting" durchgeführt, wie von Lucibello et al., EMBO J. 14, 132 (1995) und von Pfeifer et al., Science 246, 810 (1989) beschrieben.

Die folgenden Primer wurden hierzu verwendet:
1. Primer: 5'-TCAGGACTCAGGCTGCT (Seq.-ID 10)
2. Primer: 5'-CGAGCCGCTCCGGGCCCCAGG (Seq.-ID 11)
3. Primer: 5'-GGCCCCAGGCGGTGCTCTCAGGCG. (Seq.-ID 12)

Die Analyse der G0 Zellen durch genomisches "footprinting" (Fig. 3C) zeigte eine deutliche Besetzung der E2F-Bindungsstelle, sowohl des Kernelementes als auch der zwei Guanine unmittelbar stromabwärts gelegen. So waren die ♂6 Guanine in den Positionen -209, -208, -206, -205, -204 und -202 (bezogen auf das ATG Startcodon (Lam et al., EMBO J. 12, 2705 (1993)) eindeutig geschützt, wobei die zwei letzteren nur einen Teilschutz aufwiesen. Dieser Schutz gegen Methylierung war ähnlich demjenigen bei der rein biochemischen Untersuchung was die Schlußfolgerung zuläßt, daß der Proteinkomplex, der intrazellulär schützt, in der Tat E2F ist.

Ähnliche Ergebnisse konnten mit Komplexen, die E2F2, E2F3, E2F5, DP2 und DP3 enthalten, erzielt werden. Erwartungsgemäß war keine Bindung festzustellen, wenn eine Punktmutation in die E2F-Bindungsstelle eingefügt wurde (CTTGGCGG → CTTGGCTG).

Die Analyse von serumstimulierten Zellen zeigte einen ähnlichen Schutz der E2F-Bindungsequenz nach 4 Stunden. Nach 8 Stunden war dieser Schutz jedoch beträchtlich vermindert und zu späteren Zeiten konnte kein signifikanter Schutz mehr beobachtet werden. Diese Abnahme des Schutzes verlief gleichzeitig mit der Zunahme der B-myb Transkription. Im Gegensatz zum zellzyklusregulierten Schutz der E2F-Bindungssequenz vor Methylierung war beispielsweise das Guanin in der Position -223 unverändert über den gesamten Zellzyklus hypermethyliert.

Diese Ergebnisse weisen darauf hin, daß E2F-Komplexe (die zwar bei Analyse von Zellextrakten in der späten G1 und in der S-Phase gefunden werden, wie z.B. als freie E2F und E2F-Komplexe) in der lebenden Zelle, d.h. in situ, nicht an der B-myb E2F-Bindungssequenz gebunden sind.

Diese Ergebnisse belegen des weiteren, daß eine Bindung von E2F an die E2F-Bindungssequenz keine entscheidende Rolle bei der Aktivierung der Transkription des B-myb Genes spielt, da diese Bindungssequenz nicht durch E2F besetzt ist, wenn die Transkription ansteigt. Im Gegenteil: der prinzipielle Mechanismus der Wirkung von E2F zum Beispiel auf die Regulation der B-myb Expression während des Zellzyklus ist eine Hemmung der Expression in der G0- und G1-Phase.

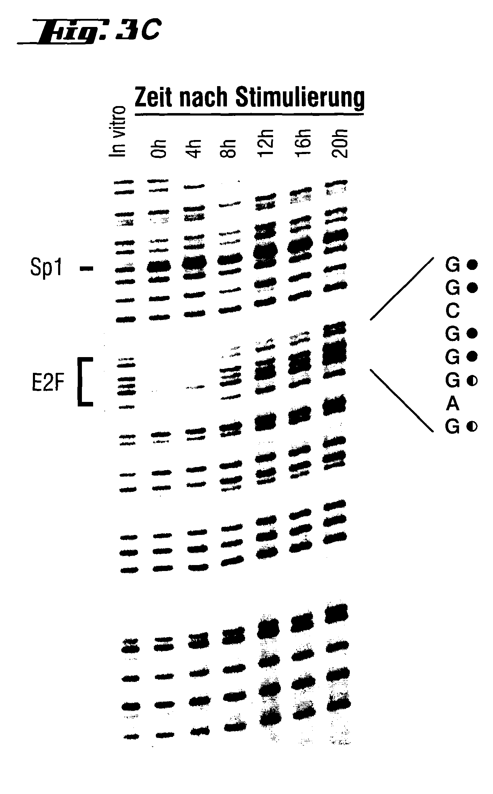

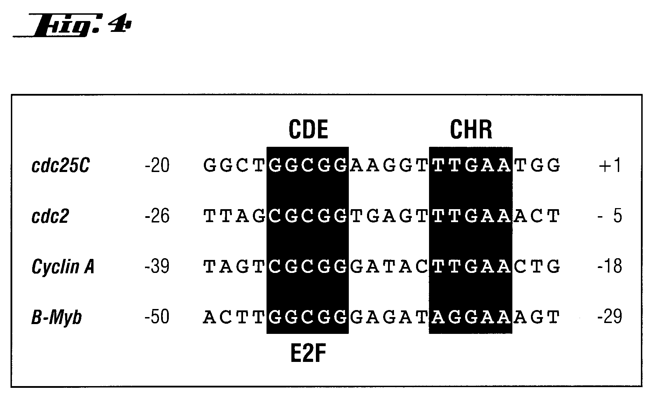

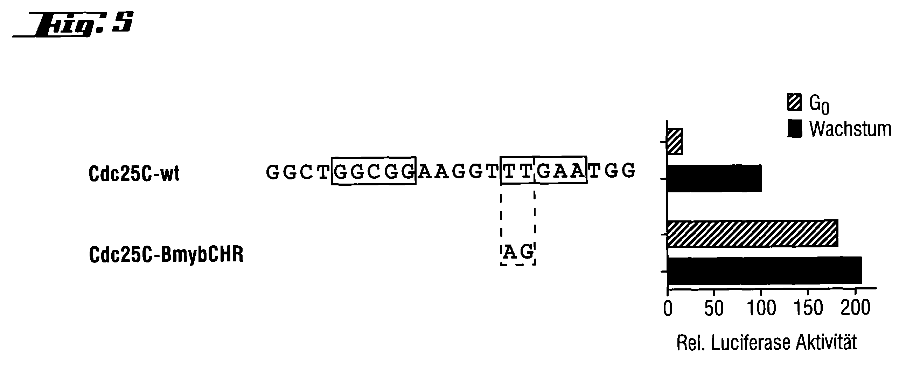

Ein Vergleich der proximalen Promotorsequenz der CDC25C, Zyklin A und CDC2-Gene mit dem B-myb-Promotor zeigte eine deutliche Ähnlichkeit nicht nur zwischen den CDEs und E2F-Bindestelle, sondern auch im Bereich der CHRs (Figur 4). In vitro-Bindungsversuche durch EMSA mit Kernextrakien aus Helazellen zeigen in der Tat, daß das B-myb-CHR von nuklearen Protein(en) erkannt wird. Aufgrund dieser Befunde wurden Konstrukte hergestellt, in denen der B-myb Promotor (Lam et al., EMBO J. 12, 2705 (1993)) an ein Luziferase-Reportergen (p x P2-Vektor beschrieben von Nordeen BioTechniques 6, 454, (1988)) gekoppelt wurde. Dieses Konstrukt zeigte, wie bei Lam et al. (1993) beschrieben, eine ca. 15-fache Repression in ruhenden Zellen (im Vergleich zu proliferierenden Zellen). Diese Repression wurde praktisch völlig zerstört (ca. 2-fache Repression) durch eine Mutation der CHR-Sequenz (TAGGAAAG → TAGGCCTG). In weiteren Experimenten wurden Konstrukte hergestellt, in denen der CDC25C-Promotor an ein Luziferase-Reportergen gekoppelt wurde. Außerdem wurde in diesem Konstrukt das CHR-Element des CDC25C-Gens gegen das CHR-Element vom B-myb-Gen ausgetauscht (Figur 5). Dieses chimäre Konstrukt war nicht mehr zellzyklusreguliert. Diese Daten belegen eindeutig, daß das B-myb-CHR und das CHR des CDC25C-Gens funktionell nicht äquivalent sind.

Nukleotidsequenzen, an welche Transkriptionsfaktoren der E2F- und CHF-Familien binden, wie beispielsweise die Nukleotidsequenz CTTGGCGGGAGATAGGAAAGT, müssen somit als neue Promotormodule angesehen werden, welche durch Bindung von E2F- und CHF-Komplexen in der G0- und G1-Phase die Transkription eines stromabwärts gelegenes Genes inhibieren.

Die Ergebnisse der Experimente werden in den anliegenden Zeichnungen näher erläutert. Die einzelnen Figuren zeigen dabei:
Figur 1
   Wechselwirkung der B-myb E2F-Bindungsstelle mit unterschiedlichen E2F-DP1 und E2F-DP1-p107 Komplexen. Komplexe wurden als rekombinante GST-Fusionsproteine hergestellt und analysiert durch EMSA (Barberis et al., Cell 50, 347 (1987)) mit Hilfe von synthetischen Oligonukleotiden, die entweder die B-myb E2F-Bindungsstelle (Lam et al., EMBO J. 12, 2705 (1993)) oder die E2F-Bindungsstelle des E2a Promotors des Adenovirus enthielten (Mudryj et al., EMBO J. 9, 2179 (1990)) Die Unterschiede in den Intensitäten, die zwischen E2F1 und E2F4 Komplexen zu sehen sind, reflektieren die Variationen in der E2F Proteinpräparationen und weniger die unterschiedlichen Bindungsaffinitäten.
Figur 2
   "Methylation protection footprints" von E2F2-DP1 und E2F4-DP1-p107 Komplexen. Methylierte Komplexe wurden mit Hilfe von EMSA (siehe Fig. 1) getrennt, die geschützte Guanine analysiert (siehe Text). Gefüllte Kreise: voller Schutz; halbgefüllte Kreise: teilweise Schutz.
Figur 3
   Kinetik der B-myb mRNA Expression und Besetzung der E2F-Bindungsstelle in der Zelle. (A) Zellzyklusanalyse von NIH3T3-Zellen, synchronisiert in G0 durch Serumentzug und stimuliert mit 10% FKS. Zellen wurden zum Nachweis der DNA mit Hoechst 33258 gefärbt und analysiert mit Hilfe des FACS (Luzibello et al., EMBO J. 14, 132 (1995)). (B) Expression von B-myb mRNA während des Zellzyklus gemessen durch RT-PCR.
   Die Isolierung der RNA und die RT-PCR wurden durchgeführt wie von Lucibello et al., EMBO J. 14, 132 (1995) beschrieben. Die Region der B-myb cDNA von Position +1630 bis +2228 wurde amplifiziert mit Hilfe der folgenden Primer: 5'-GACACCC CTGCACCAGAAGTATC und 5'-GGCTGGACTTCAGGCGGCT. GPDH diente als Kontrolle. (C) Genomisches DMS "footprinting" (Lucibello et al., EMBO J. 14, 132 (1995) and Pfeiffer et al., Science 256, 810 (1989)) der B-myb-Kern-Promotorregion in unterschiedlichen Stadien der Zellzyklusprogression. Die E2F-Bindungsstelle und eine potentiale Sp1-Bindungsstelle sind markiert. Die Analyse der Panel A, B und C wurden durchgeführt mit identischen Zellpopulationen. Gefüllte Kreise: starker Schutz (> 80%, entsprechend der "'phosphor-imaging" Analyse; halbgefüllte Kreise: teilweiser Schutz (≈ 50%)).
Figur 4
   Vergleich der proximalen Promotorsequenzen der cdc25C, Cyclin A, cdc2 und B-myb Gene. Positionsangaben sind der Literatur entnommen (Zwicker et al., EMBO J. 14, 132 (1995)) bzw. beziehen sich auf die jeweilige Hauptstartstelle (B-myb, Lam et al., EMBO J. 12, 2705 (1993)).
Figur 5
   Analyse von cdc25C-Promotor-Luziferase-Reporierkonstrukien in ruhenden (G0) und wachsenden NIH3T3-Zellen. Das Wildtyp C290 Konstrukt (Abbildung oben, Zwicker et al., 1995) wurde in CHR so mutiert (Abbildung unten, Basen unterstrichen), daß ein B-myb CHR entsteht. Die Analyse wurde durchgeführt wie in Lucibello et al., EMBO J. 14, 132 (1995) und Zwicker et al., EMBO J. 14, 4514 (1995) beschrieben.

## Patentansprüche

1. Nukleinsäurekonstrukt für die zellzyklusregulierte Expression von wenigstens einem Gen umfassend folgende Komponenten:
a) eine Aktivatorsequenz,
b) ein Promotormodul, das eine Nukleotidsequenz aufweist, die wenigstens ein Protein der E2F-Familie bindet und eine weitere Nukleotidsequenz aufweist, die wenigstens ein Protein der CHF-Familie bindet und
c) ein Gen, das für einen Wirkstoff kodiert, der zellzyklus-abhängig exprimiert wird.

2. Nukleinsäurekonstrukt nach Anspruch 1, dadurch gekennzeichnet, daß bei dem Promotormodul (b) die Nukleotidsequenz, die wenigstens ein Protein der E2F-Familie bindet, wenigstens eine Sequenz umfaßt, die ausgewählt ist aus den Nukleotidsequenzen CTTGGCGGG, TTTCGCGCC oder TTTCGCGGC.

3. Nukleinsäurekonstrukt nach einem der Ansprüche 1 oder 2, dadurch gekennzeichnet, daß bei dem Promotormodul (b) die Nukleotidsequenz, die wenigstens ein Protein der CHF-Familie bindet, wenigstens eine Sequenz umfaßt, die die Nukleotidsequenz TAGGAAA beinhaltet.

4. Nukleinsäurekonstrukt nach einem der Ansprüche 2 oder 3, dadurch gekennzeichnet, daß die Nukleotidsequenz, die CHF bindet, stromabwärts der Nukleotidsequenz für die Bindung von E2F liegt und zwischen beiden die Nukleotidsequenz AGA sich befindet (CTTGGCGGGAGATAGGAAA).

5. Nukleinsäurekonstrukt nach einem der vorhergehenden Ansprüche, dadurch gekennzeichnet, daß das Promotormodul (b) mit der stromaufwärts gelegenen Aktivatorsequenz (a) interagieren kann und dadurch die Expression des stromabwärts gelegenen Genes (c) beeinflussen, insbesondere inhibieren kann.

6. Nukleinsäurekonstrukt nach einem der vorhergehenden Ansprüche, dadurch gekennzeichnet, daß die Aktivatorsequenz zellspezifisch ist.

7. Nukleinsäurekonstrukt nach einem der vorhergehenden Ansprüche, dadurch gekennzeichnet, daß die Aktivatorsequenz virusspezifisch ist.

8. Nukleinsäurekonstrukt nach einem der vorhergehenden Ansprüche, dadurch gekennzeichnet, daß es sich bei der Nukleinsäure um DNS handelt.

9. Nukleinsäurekonstrukt nach einem der vorhergehenden Ansprüche, dadurch gekennzeichnet, daß es sich bei dem Nukleinsäurekonstrukt um einen Vektor handelt.

10. Nukleinsäurekonstrukt nach Anspruch 9, dadurch gekennzeichnet, daß es sich um einen Plasmidvektor handelt.

11. Nukleinsäurekonstrukt nach Anspruch 9, dadurch gekennzeichnet, daß es sich um einen viralen Vektor handelt.

12. Nukleinsäurekonstrukt nach einem der vorhergehenden Ansprüche, dadurch gekennzeichnet, daß es sich bei dem Gen (c) um ein Gen handelt, das für einen Wirkstoff kodiert, der ausgewählt ist aus der Gruppe umfassend Cytokine, Wachstumsfaktoren, Rezeptoren für Cytokine oder Wachstumsfaktoren, antiproliferativ oder zytostatisch wirkende Proteine, Antikörper, Antikörperfragmente, Angiogeneseinhibitoren, Gerinnungsfaktoren und Gerinnungshemmer.

13. Nukleinsäurekonstrukt nach einem der vorhergehenden Ansprüche, dadurch gekennzeichnet, daß die Aktivatorsequenz (a) ausgewählt ist aus der Gruppe von Promotoren aktiviert in Endothelzellen, glatten Muskelzellen, Makrophagen, Lymphozyten, Leukämiezellen, Tumorzellen und Gliazellen oder von Promotorsequenzen der Viren HBV, HCV, HSV, HPV, EBV, HTLV oder HIV.

14. Nukleinsäurekonstrukt nach einem der vorhergehenden Ansprüche, dadurch gekennzeichnet, daß es sich bei dem Gen (c) um ein Gen handelt, das für ein Enzym kodiert, welches eine Vorstufe eines Pharmakons in ein Pharmakon spaltet.

15. Isolierte Zelle, dadurch gekennzeichnet, daß sie ein Nukleinsäurekonstrukt gemäß einem der Ansprüche 1 bis 14 enthält.

16. Verwendung eines Nukleinsäurekonstrukts nach einem der Ansprüche 1 bis 14 oder einer Zelle nach Anspruch 15 zur Herstellung eines Heilmittels zur Behandlung einer Erkrankung, die mit Vermehrung einer Zielzelle einhergeht, wobei die Herstellung des Heilmittels die Einführung eines Nukleinsäurekonstrukts in die Zielzelle umfaßt.

17. Verwendung nach Anspruch 16, dadurch gekennzeichnet, daß es sich bei der Erkrankung, die mit übermäßiger Zellvermehrung einhergeht, um eine Tumorerkrankung handelt.

18. Verwendung nach Anspruch 16 oder 17, dadurch gekennzeichnet, daß die Herstellung des Heilmittels die Überführung eines Nukleinsäurekonstruktes nach einem der Ansprüche 1 bis 14 in eine Form umfaßt, die die Einführung des Nukleinsäurekonstruktes in die Zielzelle umfaßt.


SEQUENZPROTOKOLL

(1) ALLGEMEINE INFORMATION:

(i) ANMELDER:
(A) NAME: Hoechst Aktiengesellschaft
(B) STRASSE: -
(C) ORT: Frankfurt
(D) BUNDESLAND: -
(E) LAND: Deutschland
(F) POSTLEITZAHL: 65926
(G) TELEPHON: 069-305-3005
(H) TELEFAX: 069-35-7175
(I) TELEX: 4 1 234 700 ho d

(ii) ANMELDETITEL: Nukleinsaeurekonstrukte fuer die
zellzyklusregulierte Expression von Genen, derartige
Konstrukte enthaltende Zellen sowie deren Verwendung zur
Herstellung von Heilmitteln

(iii) ANZAHL DER SEQUENZEN: 12

(iv) COMPUTER-LESBARE FORM:
(A) DATENTRÄGER: Floppy disk
(B) COMPUTER: IBM PC compatible
(C) BETRIEBSSYSTEM: PC-DOS/MS-DOS
(D) SOFTWARE: PatentIn Release #1.0, Version #1.25 (EPA)

(2) INFORMATION ZU SEQ ID NO: 1:

(i) SEQUENZ CHARAKTERISTIKA:
(A) LÄNGE: 9 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..9

(xi) SEQUENZBESCHREIBUNG: SEQ ID NO: 1:

CTTGGCGGG 9

(2) INFORMATION ZU SEQ ID NO: 2:

(i) SEQUENZ CHARAKTERISTIKA:
(A) LÄNGE: 9 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..9

(xi) SEQUENZBESCHREIBUNG: SEQ ID NO: 2:

TTTCGCGCC 9

(2) INFORMATION ZU SEQ ID NO: 3:

(i) SEQUENZ CHARAKTERISTIKA:
(A) LÄNGE: 9 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..9

(xi) SEQUENZBESCHREIBUNG: SEQ ID NO: 3:

TTTCGCGGC 9

(2) INFORMATION ZU SEQ ID NO: 4:

(i) SEQUENZ CHARAKTERISTIKA:
(A) LÄNGE: 7 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..7

(xi) SEQUENZBESCHREIBUNG: SEQ ID NO: 4:

TAGGAAA 7

(2) INFORMATION ZU SEQ ID NO: 5:

(i) SEQUENZ CHARAKTERISTIKA:
(A) LÄNGE: 21 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..21

(xi) SEQUENZBESCHREIBUNG: SEQ ID NO: 5:

CTTGGCGGGA GATAGGAAAG T 21

(2) INFORMATION ZU SEQ ID NO: 6:

(i) SEQUENZ CHARAKTERISTIKA:
(A) LÄNGE: 42 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..42

(xi) SEQUENZBESCHREIBUNG: SEQ ID NO: 6:

GGCGCCGACG CACTTGGCGG GAGATAGGAA AGTGGTTCTG TG 42

(2) INFORMATION ZU SEQ ID NO: 7:

(i) SEQUENZ CHARAKTERISTIKA:
(A) LÄNGE: 42 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..42

(xi) SEQUENZBESCHREIBUNG: SEQ ID NO: 7:

GGCGCCGACG CACTTGGCTG GAGATAGGAA AGTGGTTCTG TG 42

(2) INFORMATION ZU SEQ ID NO: 8:

(i) SEQUENZ CHARAKTERISTIKA:
(A) LÄNGE: 30 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..30

(xi) SEQUENZBESCHREIBUNG: SEQ ID NO: 8:

GATCGACTAG TTTCGCGCCC TTTCTACTAG 30

(2) INFORMATION ZU SEQ ID NO: 9:

(i) SEQUENZ CHARAKTERISTIKA:
(A) LÄNGE: 21 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..21

(xi) SEQUENZBESCHREIBUNG: SEQ ID NO: 9:

GATCCTCTCA CCTGCTGCTA G 21

(2) INFORMATION ZU SEQ ID NO: 10:

(i) SEQUENZ CHARAKTERISTIKA:
(A) LÄNGE: 17 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..17

(xi) SEQUENZBESCHREIBUNG: SEQ ID NO: 10:

TCAGGACTCA GGCTGCT 17

(2) INFORMATION ZU SEQ ID NO: 11:

(i) SEQUENZ CHARAKTERISTIKA:
(A) LÄNGE: 21 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..21

(xi) SEQUENZBESCHREIBUNG: SEQ ID NO: 11:

CGAGCCGCTC CGGGCCCCAG G 21

(2) INFORMATION ZU SEQ ID NO: 12:

(i) SEQUENZ CHARAKTERISTIKA:
(A) LÄNGE: 24 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..24

(xi) SEQUENZBESCHREIBUNG: SEQ ID NO: 12:

GGCCCCAGGC GGTGCTCTCA GGCG 24